# EUROPEAN PATENT APPLICATION

(11) **EP 4 036 221 A1**
(43) Date of publication of application: **03.08.2022**
(21) Application number: 21154298.0
(22) Date of filing: 29.01.2021
(51) Int. Cl.: C12N 5/071, C12N 5/0775, A61K 35/39, A61K 35/407

(54) **METHOD FOR STIMULATING CELL GROWTH AND PROLIFERATION OF CELLS OF INTEREST, AND METHOD FOR PREPARING A LIVER OR PANCREAS IMPLANT**

(71) Applicant: Baer, Hans Ulrich, 8870 Freienbach (CH)
(72) Inventor: BAER, Hans Ulrich, 8870 Freienbach (CH); SIUFUI, Hendrawan, 8870 Freienbach (CH)
(74) Representative: Schaad, Balass, Menzl & Partner AG

(57) **Abstract**

The present invention provides inter alia a method for preparing a liver or pancreas implant including the steps of:
2a) providing a carrier matrix suitable for tissue engineering and a cell suspension comprising viable hepatocytes and/or cells of islets of Langerhans in a standard culture medium A;
2b) loading the cell suspension onto the carrier matrix;
2c) incubating the loaded matrix for a first incubation time T1 of at least 10 hours at about 37°C in either
- standard culture medium A or
- a culture medium B comprising standard culture medium A and 0.5% to 5% secretome;

2d) exchanging the culture medium of step 2c) with culture medium B comprising standard culture medium A and 0.5% to 5% secretome;
2e) incubating the loaded matrix for a second incubation time T2 of at least 24 hours at about 37°C in culture medium B;
2f) washing the loaded matrix of step e) with standard culture medium A to remove at least part of the secretome.

## Description

The present invention relates to a method for stimulating cell growth and proliferation of cells of interest, specifically hepatocytes and/or cells of islets of Langerhans, according to Claim 1 and to a method for the preparation of a liver or pancreas implant according to Claim 2.

Every day people die due to organ failure. Unfortunately, patients currently suffering from conditions leading to organ failures have limited treatment options. Even though conventional drug therapy may help to delay or reduce the symptoms of disease, it is often unable to restore complete functionality of the damaged tissue. For that reason, in case of end-stage organ failure, transplantation of a healthy organ or at least of some healthy organ tissue from a suitable tissue donor is the ultimate treatment option for many patients.

For instance, transplantation of healthy liver or pancreatic tissue has proven successful in many patients with severe pancreatic or hepatic diseases. However, the lack of suitable tissue donors remains a major obstacle. In an attempt to find alternative treatment methods, cell-based therapies have been gaining importance. In these cell-based therapies, tissues from the patient are extracted, processed to viable single cells and cultivated in order to obtain a large viable cell population. The cells are then directly transplanted to facilitate the body's natural regenerative processes and contribute to regeneration of failing organs or damaged tissues.

In the therapeutic field of hepatic diseases, hepatocyte transplantation has gained importance as alternative or supportive treatment to orthotopic liver transplantation for congenital or fulminant liver disease. However, difficulties in cultivation of hepatocytes (meaning harvesting cells from a patient's own tissue to be grown and expanded in the laboratory) and limited long-term survival of the transplanted cells present major obstacles for broader application of this cellular approach.

A newer interesting cell-based approach is hepatocyte and pancreatic islet co-transplantation: It has been found that proliferation and survival of hepatocytes can be stimulated by co-cultivation with cells of islets of Langerhans (or short: "islet cells"). This concept is described e.g. in EP 3 010 556 B1. Particularly, the beta cells (insulin producing cells) were found to increase the growth rate of hepatocytes up to 300%. On the downside, this means that the production of an autologous liver implant (i.e. a carrier matrix loaded with viable hepatocytes) requires not only an extraction of liver tissue from a patient but also a separate extraction of the patient's pancreatic tissue by open or laparoscopic operation. The extracted tissues are then processed outside the patient's body to harvest both hepatocytes and cells of islets of Langerhans for co-cultivation. If a sufficiently high number of hepatocytes and cells of islets of Langerhans could be cultivated in-vitro, a second operation is performed, in which one or more suitable carriers loaded with the cultivated cells are inserted below the serosa of the small bowel mesentery or other suitable locations of the patient. However, every operation carries a certain risk and in particular in pancreatic tissue removal there is a considerable risk of an acute inflammation (pancreatitis) or damaging the pancreatic duct that can lead to dangerous or even life-threatening pancreatic juice fistula formation. Methods are therefore being sought which could stimulate the hepatocyte growth - ideally without the need of co-cultivation with pancreatic cells.

It is therefore an object of the present invention to provide a safer and less time and cost-intensive method for the cultivation cells, in particular of hepatic and/or pancreatic cells, in view of their use in the field of regenerative medicine. More specifically, the present invention aims to provide a method for safe and cost-efficient preparation of an implant in general, and in particular a liver or pancreas implant.

This object is achieved by the methods of Claims 1 and 2. Preferred embodiments are subject to the dependent claims.

The invention described herein encompasses a method for stimulating cell growth and proliferation by incubation of the cells of interest in a cell growth medium or fluid together with secretome derived from mesenchymal stem cells. Specifically, the cells of interest (or "target cells") are hepatocytes (including hepatocyte progenitor cells) and/or cells of islets of Langerhans, i.e. pancreatic cells. For both, hepatocytes and cells of islets of Langerhans, incubation together with secretome has proven particularly effective to promote proliferation and enhance the survival rate of these cells.

In particular, in one aspect the present invention provides a method for stimulating cell growth and proliferation of cells of interest, specifically hepatocytes and/or cells of islets of Langerhans, by performing the following steps:
1a) providing viable cells of interest;
1b) incubating the cells of interest for a first incubation time T1 of at least 10 hours at about 37°C in either
   - a standard culture medium A or
   - a culture medium B comprising standard culture medium A and 0.5% to 5% secretome;
1c) exchanging the culture medium of step 1b) with culture medium B comprising standard culture medium A and at least 0.5% to 5% secretome; and
1d) incubating the cells of interest in the culture medium B for a second incubation time T2 of at least 24 hours.

The present invention is based on the inventor's discovery that not only the presence of mesenchymal stem cells (MSC) but also the secretome of MSC can positively affect the proliferation of cells, even sensitive cells such as hepatocytes, in vitro and in vivo. Within the context of the present application the term "secretome" refers to factors released from mesenchymal stem cells (MSC), which are found in bone marrow, muscle, and adipose tissue, and also in Wharton's jelly, dental pulp, peripheral blood, skin, lungs, chorionic villi, menstrual blood, and breast milk. It was found that the inventive method involving cultivation of cells of interest in a culture medium containing 0.3% to 5% MSC secretome for at least 24 hours significantly increased the proliferation and survival rate of the cells.

It was surprisingly found that the stimulating effect on proliferation and survival was also present for hepatocytes: It is well-known that the function of isolated primary hepatocytes is hard to maintain when cultured in vitro, as these cells readily undergo dedifferentiation, causing them to lose hepatocyte function. However, developing a sufficient number of functional hepatocytes is a prerequisite for treatment of liver diseases that result in terminal hepatic failure and therefore require liver cell transplantation and artificial liver support systems.

The inventors of the inventive methods described herein were thus very excited about the finding that a treatment of hepatocytes in accordance with the method of the present invention enhanced the growth and proliferation rate of the hepatocytes at least as much or even higher than when the hepatocytes were co-cultured with cells of islets of Langerhans (as mentioned above, the latter being so far one of the most effective known hepatocyte stimulation methods). The method of the present invention therefore provides a new way to stimulate cell growth and proliferation of hepatocytes without needing to co-cultivate them with cells of islets of Langerhans. In practice, this means that the production of an autologous liver implant or an autologous liver cell transplantation can be achieved by extraction of autologous hepatocytes, cultivation and re-implantation thereof without the need for an additional surgical procedure for extraction of cells of islets of Langerhans (i.e. pancreatic cells) from the patient for co-cultivation with the hepatocytes.

As promising as these new findings were, some new challenges had to overcome if MSC secretome was to be used for stimulating cell growth in a clinical context: it was found that some factors in the MSC secretome - depending on the concentration and release kinetics - can also exert inhibitory effects on the microenvironment, such as apoptosis of cells, inflammation, pathological remodelling, or scar formation. Also, the growth-stimulating effects exhibited by MSC secretome could not be reproduced (or at least not to the same degree) by the delivery of any of the identified cytokines/growth factors in isolation.

Therefore, when using MSC secretome to stimulate cell growth in vivo, an appropriate balance between stimulatory and inhibitory factors produced by MSC must be accomplished, and the desired effects must be preserved following in vivo transplantation. The present invention has found a way to overcome these challenges and presents a method that allows for safe use of MSC secretome in the preparation of a tissue implant for later use in regenerative medicine.

Specifically, in a second aspect, the present invention provides a method for preparing an implant for use in regenerative medicine including the steps of:
2a) providing a carrier matrix suitable for tissue engineering and a cell suspension comprising viable hepatocytes in a standard culture medium A;
2b) loading the cell suspension onto the matrix;
2c) incubating the loaded matrix for an incubation time T1 of at least 10 hours at about 37°C in either standard culture medium A or a culture medium B comprising standard culture medium A and at least 0.5% secretome;
2d) exchanging culture medium of step 2c) with culture medium B comprising standard culture medium A and at least 0.5% secretome;
2e) incubating the matrix for an incubation time T2 of at least 24 hours at about 37°C in culture medium B;
2f) washing the hepatocyte-loaded matrix of step f) with standard culture medium A to remove at least part of the secretome.

The methods in accordance with the present invention involve use of a "standard culture medium A". Incubation of the cells can take place in any appropriate cell growth medium with incubation conditions that supports growth, differentiation and protein synthesis of the cells of interest. Many commercially available culture media, such as Dulbecco's Modified Eagles Medium (DMEM), William E, RPMI 1640, Fisher's, Iscove's, and McCoy's, may be suitable for use as standard culture medium A. The medium may be supplemented with additional salts, carbon sources, amino acids, serum and serum components, vitamins, minerals, reducing agents, buffering agents, lipids, nucleosides, antibiotics, attachment factors, and growth factors. Formulations for different types of culture media are described in various reference works available to the skilled person (e.g. Culture of Animal Cells, A Manual of Basic Techniques, 4th Ed., Wiley-Liss (2000)). The standard culture medium used in any of the culturing steps of the present invention - whether under aerobic (normoxic) or hypoxic conditions - may include serum, or be serum free. A preferred standard culture medium is "Williams E Medium", which is widely used in cell culture laboratories and well known to the skilled person.

The term "matrix" as used therein refers to a scaffold of three-dimensional architecture. The three-dimensional support or scaffold used to culture cells may be of any material and/or shape that: (a) allows cells to attach to it (or can be modified to allow cells to attach to it); and (b) allows cells to grow in more than one layer (i.e., form a three-dimensional tissue). In this sense, the matrix serves as a three-dimensional template which can be colonized by cells or tissue. This colonization can take place in vitro or in vivo. Furthermore, the matrix serves, in connection with transplantations, for locating the transplant and also as a place holder for tissue which is gradually formed in vivo. The matrix preferably has a sponge-like structure with at least partly interconnected pores of different pore sizes.

Independent of the use of a matrix as carrier, a key aspect of the present invention is the cultivation of the cells of interest - in particular hepatocytes and/or cells of islets of Langerhans - in a culture medium B comprising secretome. In particular, it is preferred that culture medium B comprises secretome derived from umbilical cord mesenchymal stem cells (hucMSC). Similar to the MSC from bone marrow, human umbilical cord-MSCs (hucMSC) have a high self-renewal ability and low immunogenicity, and hucMSC can be obtained by a non-invasive procedure and easily cultured, which make them potentially superior to the MSC from other sources.

MSC secretome can either be provided as MSC-conditioned media (CM) or purified MSC-derived extracellular vesicles (EVs). Surprisingly, a particularly high proliferation and survival rate was found if the cells, in particular hepatocytes, were seeded in conditioned media from MSC grown under hypoxic conditions, instead of using conditioned media from the same cells grown under normoxic conditions. As used herein, hypoxic conditions are characterized by a lower oxygen concentration as compared to the oxygen concentration of ambient air (ambient air contains approximately 15%-20% oxygen). Preferably, the hypoxic conditions are at 3%-5% oxygen. Hypoxic conditions can be created and maintained by using a culture apparatus that allows one to control ambient gas concentrations, for example, an anaerobic chamber.

Without wanting to be bound by the theory it is assumed that hypoxic exposure promotes self-renewal of undifferentiated mesenchymal stem cells and enhances their therapeutic potential by activation several signal transduction pathways including hypoxia inducible factor (HIF), a master transcription factor that regulates the ex-pression of hundreds of genes to promote cellular adaptation to the hypoxic condition.

Thus, for the methods of the present invention it is preferred that the secretome is derived from mesenchymal stem cells, preferably umbilical cord mesenchymal stem cells, cultured under hypoxic conditions.

Preferably, commercially available secretome from human Wharton's jelly stem cells is used. This secretome was found to be particularly effective for stimulating hepatocytes.

With respect to the concentration of the secretome in culture medium B it was found that the best growth stimulation of cells in general and in particular of hepatocytes occurred when using secretome from MSC, preferably from human Wharton's jelly stem cells, at a concentration of 0.5% to 5%, preferably 0.5% to 3%, more preferably about 1%. At least for hepatocytes, higher concentrations than 5% secretome in the culture medium B did not favour but actually decreased the surviving rate.

For both methods of the present invention - i.e. for providing cell growth and proliferation of cells of interest in general and in particular of hepatocytes and/or cells of islets of Langerhans - and for providing an implant for use in regenerative medicine it is preferred that the first incubation time T1 is 17 to 27 hours, preferably about 24 hours.

The second incubation time T2 is generally longer than the first incubation time T1 and is preferably within the range of 35 to 50 hours, preferably about 48 hours.

These incubation times were found to be most effective for obtaining a large cell population and - in case of preparing an implant - achieving a high adherence rate of cells to the matrix. In particular for the growth of hepatocytes, the first incubation time is about 24 hours and the second incubation time is about 48 hours, since the sensitive hepatocytes generally need more growth and stimulation time.

Although it is generally preferred to load the matrix with the cells of interest prior to incubating the cells in the secretome containing culture medium B, cultivation of the cells of interest in the presence of MSC secretome can also occur before being transferred a matrix as carrier. In view of using the cell loaded matrix as an implant, the method of the present invention requires that both the matrix and the cells loaded thereon are subjected to a cleaning treatment to remove the secretome. The resulting implant can then be re-implanted into the body to take over the function of a damaged organ within the patient.

Animal studies confirmed that cells cultivated in the presence of MSC secretome and treated in line with the methods of the present invention maintained an enhanced cell survival rate and improved homing to their target site after in-vivo transplantation. Since the secretome is at least partly removed from the implant before its implantation, there are no concerns about possible negative or inhibitory effects of the secretome in the patient's body. Apart from potential immune rejection and pathogen transmission if a non-allogenic secretome is used, the removal of the secretome from the cell-loaded matrix also overcomes regulatory issues associated with transplantation of stem cells or stem cell secretome.

Thus, when e.g. applied to the production of a liver implant, the method of the present invention allows for a safer and less cost-intensive way to produce an implant with a large viable population of hepatocytes or hepatocyte-like cells compared to the standard method involving extraction and co-cultivation of hepatocytes and cells of islets of Langerhans.

In a preferred embodiment, in step 2c) of the inventive implant preparation method, the loaded matrix is first incubated in standard culture medium A for about 30 min prior to incubating the matrix in culture medium B for the incubation time T1. This has shown to further increase the attachment rate of the cells. This pre-treatment step is also preferable for the method defined in Claim 1 of this application.

As regards the washing step 2g), it is preferred that the washing is conducted by a first culture medium exchange step in which culture medium B is exchanged with fresh standard culture medium A. This first culture medium exchange step is preferably followed by at least a second culture medium exchange step in which previously added standard culture medium A is exchanged by fresh standard culture medium A.

In a particularly preferred embodiment, the washing step 2g) is conducted as described in the previous paragraph and more specifically such that the first and/or second culture medium exchange step(s) is/are conducted by
i) transferring the matrix into a new container comprising fresh standard culture medium A and
ii) repeating step i) at least once, preferably at least twice, most preferably three times.

Instead of placing the matrix into a new container with fresh standard culture medium A, the matrix may also remain in the initial container while the culture medium is carefully aspirated, e.g. by using a pipette, and replaced with fresh medium that is carefully added around the matrix.

This preferred way of washing the secretome-treated, cell loaded matrix has proven to be particularly gentle and thus very well suited for sensitive cells, such as hepatocytes.

To allow not only proper cell growth but also removal of the secretome, the matrix preferably includes a porous scaffold, preferably a sponge-like structure with at least partly interconnected pores. Porous scaffolds refer to mesh structures having pores through which molecules may diffuse in or out. The porous nature of the matrix facilitates attachment of the cells and nutrient flow through the matrix.

With respect to its composition, the matrix may be chosen depending on the tissue to be treated, type of damage to be treated, the length of treatment desired, longevity of the cell culture in vivo, and time required to prepare the matrix. The matrix may comprise various polymers, natural or synthetic, charged (i.e. anionic or cationic) or uncharged, biodegradable, or nonbiodegradable.

The expression "biodegradable" refers to a material which can be converted into metabolizable products in living organisms (or body fluids or cell cultures derived from living organisms). Biologically degradable materials include, for example, polymers which are bioresorbable and/or bioerodable. "Bioerodable" denotes the ability to be soluble or suspendable in biological liquids. Bioresorbable means the ability to be able to be taken up by cells, tissues or fluids of a living organism.

Degradable polymers include naturally occurring polymers, such as fibrin, casein, serum albumin, collagen, gelatin, lecithin, chitosan, alginate or poly-amino acids such as poly-lysine. Alternatively, the matrix may be made of synthetic polymers, non-limiting examples of which include, among others, polylactide (PLA), polyglycolide (PGA), poly(lactide-co-glycolide) (PLGA), poly(caprolactone), polydioxanone trimethylene carbonate, polyhybroxyalkonates (e.g., poly(hydroxybutyrate), poly(ethyl glutamate), poly(DTH iminocarbony(bisphenol A iminocarbonate), poly(ortho ester), and polycyanoacrylates.

In a preferred embodiment, the matrix consists of one or more polymers selected from the group consisting of PLA, PGA, mixtures thereof (PLGA) and a natural polymer, such as collagen. A possible basic polymer material for the matrix is a 50:50 mixture of PLA and PGA - i.e. a polymer mixture having a lactic acid (PLA) content of about 50 mol% and a glycolic acid (PGA) content of about 50 mol%. Such a 50:50 mixture can be purchased, for instance, from Evonik Industries AG (Essen, Germany) under the brand name RESOMER^{®} RG 502, or from Durect company (Cupertino, CA, USA) under the brand name Lactel B6007-1.

Preferably, however, the matrix includes a basic polymeric structure that mainly or completely consists of PLA, in particular PLLA (e.g. Lactel B6002-2). With "mainly" it is meant that the basic polymeric structure preferably consists to at least 85%, preferably at least 90% of PLA. Said basic polymeric structure is preferably covered by a natural polymer, in particular collagen.

Preparation methods for preparing a matrix from the above-mentioned synthetic polymers are well known in the art. One possibility is the use of a salt-leaching technique, as described for instance in EP 2256155.

It is further preferred that the matrix comprises, or is at least partially covered with, at least one natural polymer selected from the group consisting of collagen, gelatin, laminin, fibrinogen, albumin, chitin, chitosan, agarose, hyaluronic acidalginate and mixtures thereof, whereby collagen is preferred. The natural polymer provides the matrix with additional stability, hydrophilicity and facilitates cell adhesion and proliferation. A matrix with a collagen coating can be obtained, for instance, by covering the matrix with or soaking the matrix in a 1% collagen solution, followed by freeze-drying.

In some embodiments, the matrix may comprise hydrogels, which are typically hydrophilic polymer networks filled with water. Hydrogels have the advantage of selective trigger of polymer swelling. Depending on the composition of the polymer network, swelling of the matrix may be triggered by a variety of stimuli, including pH, ionic strength, thermal, electrical, ultrasound, and enzyme activities. Non-limiting examples of polymers useful in hydrogel compositions include, among others, those formed from polymers of poly(lactide-co-glycolide); poly(N-isopropylacrylamide); poly(methacrylic acid-g-polyethylene glycol); polyacrylic acid and poly(oxypropylene-co-oxyethylene) glycol; and natural compounds such as chrondroitan sulfate, chitosan, gelatin, fibrinogen, or mixtures of synthetic and natural polymers, for example chitosan-poly (ethylene oxide). The polymers may be crosslinked reversibly or irreversibly to form gels adaptable for forming the matrix.

To facilitate cell attachment and growth throughout the matrix, the latter is preferably hydrophilic, at least at its surface. The term "hydrophilic" or "hydrophilicity" as used in the context of the present invention refers to a water contact angle of a surface area on the matrix being below 75°. A hydrophilic surface can be obtained for example by increasing the content of - or covering the matrix with - hydrophilic polymers, such as gelatin or collagen, in the matrix material and/or by post-processing methods, such as plasma-treatment. The term "plasma" thereby generally refers to an excited and radicalized gas, i.e. an electrical conducting process gas involving electrons and ions. Plasma is commonly generated by means of electrodes in a vacuum chamber (so-called "RF plasma approach"), but it can also be generated using capacitive or inductive methods, or microwave radiation. More details in this respect are found e.g. in WO 2017/032837, which contents is hereby incorporated by reference.

In view of its later use as an implant, the matrix is preferably sterilized before being seeded with the cells of interest. To this end, the matrix is preferably subjected to a hydrogen peroxide containing environment at a temperature between 40°C to 50°C or lower.

The hydrogen peroxide containing environment can either be provided by a H₂O₂ plasma treatment or by placing the matrix to be sterilized into a vacuum chamber, together with a source of hydrogen peroxide, preferably a 30% H₂O₂ solution. Upon application of a vacuum inside the vacuum chamber, the hydrogen peroxide evaporates and a hydrogen peroxide-containing atmosphere will be created. This sterilization technique has been developed by the inventors of this application and allows sterilization of heat and/or UV sensitive tissues, in particular polymeric scaffolds, without compromising their hydrophilic properties or damaging the polymeric structure. It is therefore particularly well suited for sterilizing the matrix for use in the method of the present invention.

The treatment time of the matrix within the H₂O₂ containing atmosphere is at least 1 minute, preferably at least 5 minutes, more preferably 5 to 20 minutes, at a temperature between 40°C and 50°C. Alternatively, if the sterilization is conducted at a temperature below or at 40°C, the treatment time is preferably at least 4 hours, more preferably about 8 to 12 hours, most preferably for about 10 hours.

In addition, the negative pressure applied in the vacuum chamber is preferably in the range of 6 to 12 mbar, more preferably 8 mbar to 10 mbar, most preferably about 9 mbar.

To enhance the hydrophilicity and thus the attachment rate of the cells, the matrix is preferably is plasma-treated before being seeded with cells. The plasma treatment is preferably conducted by exposing the matrix to a reactive gas plasma for at least 1 minute, preferably at least 5 minutes, more preferably about 10 minutes, at a temperature below 50°C. For the plasma treatment, the pressure is preferably in the range of 10⁻² to 10⁻⁶ bar, more preferably within the range of 0.1 to 1.0 mbar

The matrix is preferably provided in a shape that is sheet-like, i.e. having a thickness that is smaller than the length and width of the matrix. Preferably, the matrix is also elastically deformable to allow folding or rolling thereof. This way, the matrix with the cells adhering thereto can be introduced into the body through a laparoscopic tool, e.g. a trocar. The outer shape (when seen in top view or in longitudinal section) of the matrix can be of any kind, for example rectangular, square, circular, oval, etc., and can also be cut to the desired shape. Preferably, the outer shape of the cross-section is circular or oval to avoid any sharp edges.

In view of using the matrix seeded with cells as an implant it is preferred that the matrix is loaded with at 250'000 cells, preferably at least 500'000 cells, per cm³.

For use as an implant, the matrix is preferably loaded with at least 1x10⁶ cells. For a liver implant, for instance, the matrix is preferably loaded with at least one million hepatocytes.

In a further aspect, the present invention also relates to a method for treatment of a patient in need of a liver transplant comprising the steps of
i. harvesting of liver tissue of the patient through an open or laparoscopic surgical procedure,
ii. treatment of the liver tissue in the laboratory to single out hepatocytes,
iii. seeding the hepatocytes on a matrix, preferably a matrix consisting of poly-glycolic-acid (PGA), polylactic-acid (PLA), mixtures thereof (PLGA), and collagen,
iv. stimulating the hepatocytes by incubating them in a culture medium comprising at least 0.5% MSC-derived secretome for at least 24 hours prior or after seeding the hepatocytes on the matrix,
v. washing the matrix with the attached hepatocytes with standard culture medium to remove at least part of the secretome;
vi. implanting the washed, hepatocyte loaded matrix implant into the patient's body, preferably in the small bowel mesentery.

Prior to being loaded with hepatocytes, the matrix is preferably is plasma-treated and/or sterilized with the aid of H₂O₂ as described in the sections above.

### Experimental

### Materials

### Hepatocytes and Islet Cells

Freshly isolated from either rats or from cirrhotic liver tissue / pancreatic tissue of a human patient.

### Secretome

Conditioned medium (CM) derived from human umbilical cord cultured in normoxia and hypoxia condition was obtained from Stem Cell and Cancer Institute, PT. Kalbe Farma, Tbk., Jakarta, Indonesia.

### Methods

### Preparation of standard culture medium A:

HEPES was added to William's E Medium in an amount to obtain an end concentration of 50 mM. Then, L-glutamine and sodium pyruvate was added before adding Fetal Bovine Serum (FBS) in an amount of 10% of total volume FBS.

Finally Antibiotic/Antimycotic was added in an amount of 1% of total volume. The resulting culture medium was mixed thoroughly.

| **Solution/medium** | **Description** |
|---|---|
| Williams' E Medium | Sigma; Cat. No. W4128 |
| HEPES | Sigma; Cat. No. H0887 |
| Sodium Pyruvate | Sigma; Cat. No. S8636 |
| Fetal Bovine Serum (FBS) | Sigma; Cat. No. F2442 |
| L-glutamine | Sigma; Cat. No. G7513 |
| Antibiotic/Antimycotic | Sigma; Cat. No. A5955 |

### Preparation of culture medium B containing 1% Secretome

2 mL of Secretome was added to 198 mL of standard culture medium A prepared as described above and mixed thoroughly.

### Matrix Preparation from PLLA

The PLLA matrices were prepared in accordance with the common salt leaching technique using sodium chloride (NaCl) particulates as a porogen. NaCl particulates were sieved, and particulates ranging in diameter from 355 µm to 425 µm were used. PLLA (1 g) was dissolved in chloroform (5-5.3 mL) and the sieved NaCl particulates (9.0 g) were placed in an aluminum pan. The PLLA solution was poured into the aluminum pan and mixed with the NaCl particulates. The mixture was air-dried in an air draft for 48 h. The aluminum pan was then torn and peeled off from the dried PLLA/NaCl block. The PLLA/NaCl block was dried in a vacuum for an additional 48 h and the NaCl particulates were leached out through immersion of the dried PLLA/NaCl block in deionized water, which was changed every hour. This washing by immersion continued until the weight of the PLLA sponge remained constant. Once the PLLA sponge dried completely, preparation of the PLLA matrices was complete.

### Matrix Coating with Collagen

Matrices prepared from PLLA were cut to the desired size and immersed in 1% collagen solution (Bovine derived Atelocollagen (KOKEN, Japan, IPC-50)) to fill all pores in the PLLA matrices with collagen solution to guarantee an even coating.

After complete immersion, the matrices were removed from the collagen solution, placed in a centrifuge tube and centrifuged for 5 minutes on each side (i.e. totally 10 minutes) at 1000 g, 4°C (in a Low Speed Refrigerated Centrifuge (Tomy, AX501)) to remove any excess collagen solution. The collagen-covered matrices were then put a deep freezer (New Brunswick, Innova U101) at -80°C for at least 4 hours before being freeze-dried under vacuum below 5 Pa (in a Vacuum Freeze Dryer (EYELA, FDU-2200)) for at least 24 hours. Said final freeze-drying step preserved the hydrophilicity of the coated collagen layer.

The collagen coated matrices were stored in a desiccator until use.

### Matrix Plasma Cleaning

Collagen-coated matrices prepared as described above were further cleaned by plasma cleaning using an oxygen-containing gas plasma. The matrices were placed in a vacuum chamber of a Plasma Machine (Company Diener) and vacuum chamber was degassed prior to introducing oxygen gas plasma into it at a pressure of 0.4 mbar, treatment time was between 5 and 10 minutes. This plasma treatment was found to not only clean the matrix surface but also to activate it, thereby increasing the surface's hydrophilicity.

### Matrix Sterilization

The plasma-cleaned matrices were sterilized by placing them in a H₂O₂-containing environment for at least 10 minutes (at temperatures between 40°C and 50°C) or up to 12 hours at temperatures below 40°C. The H₂O₂-containing environment was created within a vacuum chamber, by placing the matrix in a Tyvek bag into the chamber, together with an open flask containing a 30% H₂O₂ solution (from Merck, Darmstadt, Germany, #108597) and by subsequently evacuating the chamber to sublimate the H₂O₂. The treatment time depends highly on the pressure within the chamber and the concentration of the H₂O₂ solution. Preferred treatment times are between 5 minutes and 20 minutes for temperatures around 40°C and between 8 to 12, preferably about 10 hours, for temperatures below 40°C.

### Cell isolation

Perfused liver tissue was cut into small pieces and pressed through a tea strainer (1 mm mesh) using a spatula. The strainer was washed with C/H solution and the collected cell suspension was passed through a 100 micron cell sieve before being centrifuged at 130 G for 10 min. Supernatant was aspirated using a Pasteur pipette and the pellets were resuspended in Williams E medium (Williams E Med complete with 10% FBS). Centrifuging and resuspension steps were repeated several times. The resulting liver cell suspension was incubated at 37°C and 5% CO2 until further use. Cell numbers and viability was determined using Tryphan blue.

Cell mixture analysis was performed in 3D format (on collagen coated PLLA matrix).

The same procedure was applied to pancreatic tissue to obtain a cell suspension of cells of islets of Langerhans.

### Sample preparation

5 sample groups were prepared. Each group included 3 PLLA-matrices coated with 1% collagen. The matrices were plasma-treated and sterilized in accordance with the methods described above prior to being seeded with cells by adding 600-700 µL cell suspension (either hepatocytes or a mixture of hepatocytes and islet cells in a ratio of 1x10⁶ hepatocytes to 50'000 cells of islets of Langerhans) in standard culture medium A onto each of the matrices (cells density: 1,000,000 cells/matrix).
Group 1: Hepatocytes and secretome
Group 2: Hepatocytes and secretome obtained from MSC grown under hypoxic conditions
Group 3: Hepatocytes, cells of islets of Langerhans and secretome
Group 4: Hepatocytes only (Control 1)
Croup 5: Hepatocytes and cells of islets of Langerhans (Control 2)

### Sample treatment

For the control Groups 4 and 5, the matrices seeded with hepatocytes (Group 4) or hepatocytes and islet cells (Group 5) were incubated on the matrices in standard culture medium A without secretome (no addition of culture medium B).

For Groups 1, 2 and 3: The matrices with the cells seeded thereon incubated at 37°C and 5% CO₂ for 30 min. Then, standard culture medium A around the matrices was aspirated using a micropipette and 600 µL culture medium B comprising 1% secretome was added to the matrices under sterile conditions. The cells in the aspirated medium were counted using a haemacytometer (manual counting).

On day 2 (after 24h), under sterile conditions, any leftover medium was aspirated under sterile conditions and 1 mL of fresh culture medium B was added.

The cells in the aspirated medium were again counted using a haemacytometer (manual counting).

### Secretome wash-out procedure

For all matrices, including control groups, the following washing procedure was performed on day 4 (63-87 h after seeding):
The matrices were transferred into new wells and 1 mL of fresh standard culture medium A was added dropwise around the matrices using a micropipette to wash out the secretome. This procedure was performed repeated 3 times.

After transferring the matrices into new wells the number of cells that remained in the former wells were determined by manual counting.

The adhesion rate was determined using the formula below:
Number of cells placed on the matrices - number of cells remaining in the wells = number of adherent cells on the matrix.

### Results

While further studies are still running, the above-described in-vitro study showed the following results:

| Sample Group | Adherence Rate |
|---|---|
| Group 1 (Hep. & Secretome) | 65% |
| Group 2 (Hep. & Secretome Hypoxia) | 70% |
| Group 3 (Hep. & cells of IL & Secretome) | 74% |
| Group 4 (Hep. only) | 39% |
| Group 5(Hep. & cells of IL) | 63% |

The above numbers show the following order in terms of adherence rates:
Group 3 > Group 2 > Group 1 ≈ Group 5 > Group 4

Highest adherence rate of was found for Group 3, i.e. for matrices seeded with hepatocytes and cells of islets of Langerhans and treated with 1% secretome. Group 2 (hepatocytes and secretome derived under hypoxic conditions) Groups 1 (Hepatocytes and Secretome) and Group 5 (control group with matrices seeded with hepatocytes and cells of islets of Langerhans) showed similar adherence rates, but were still clearly higher than the matrices of control Group 4 seeded with only hepatocytes.

The conducted in-vitro experiment showed that MSC secretome was able to increase the viability and adhesion rate of hepatocytes on matrices significantly. Cultivation of hepatocytes in culture media containing MSC secretome led to better results with respect of adhesion rate and proliferation than co-cultivation of hepatocytes and islet cells. Best stimulation was achieved by combined co-cultivation with cells of islets of Langerhans in culture media containing MSC secretome.

While the addition of secretome derived from MSC under normal and hypoxic conditions both incresed the adherence rate, secretome derived from MSC under hypoxic conditions was more beneficial.

An important finding was that matrices seeded with hepatocytes and treated with secretome derived from MSC cultured under normal conditions showed a similar - or even slightly better - adherence rate as matrices seeded with a mixture of hepatocytes and cells of islets of Langerhans (without secretome addition). Matrices seeded with hepatocytes and treated with secretome derived from MSC cultured under hypoxic conditions were even clearly superior in terms of adherence rates. This means that the addition of secretome, in particular secretome obtained under hypoxia condition, is a valid alternative to co-seeding with cells of islets of Langerhans to stimulate cell growth and adhesion of hepatocytes. This alternative has the advantage that no pancreatic tissue needs to be harvested for the production of a liver implant.

## Claims

1. Method for stimulating cell growth and proliferation of cells of interest, specifically hepatocytes and/or cells of islets of Langerhans, by performing the following steps:
1a) providing viable cells of interest;
1b) incubating the cells of interest for a first incubation time T1 of at least 10 hours at about 37°C in either
- a standard culture medium A or
- a culture medium B comprising standard culture medium A and 0.5% to 5% secretome;
1c) exchanging the culture medium of step 1b) with culture medium B comprising standard culture medium A and 0.5% to 5% secretome; and
1d) incubating the cells of interest in the culture medium B for a second incubation time T2 of at least 24 hours.

2. Method for preparing a liver or pancreas implant including the steps of:
2a) providing a carrier matrix suitable for tissue engineering and a cell suspension comprising viable hepatocytes and/or cells of islets of Langerhans in a standard culture medium A;
2b) loading the cell suspension onto the carrier matrix;
2c) incubating the loaded matrix for a first incubation time T1 of at least 10 hours at about 37°C in either
- standard culture medium A or
- a culture medium B comprising standard culture medium A and 0.5% to 5% secretome;
2d) exchanging the culture medium of step 2c) with culture medium B comprising standard culture medium A and 0.5% to 5% secretome;
2e) incubating the loaded matrix for a second incubation time T2 of at least 24 hours at about 37°C in culture medium B;
2f) washing the loaded matrix of step e) with standard culture medium A to remove at least part of the secretome.

3. Method according to Claim 1 or 2, wherein the culture medium B comprises secretome derived from umbilical cord mesenchymal stem cells.

4. Method according to Claim 3, wherein the secretome is derived from umbilical cord mesenchymal stem cells cultured under hypoxic conditions.

5. Method according to any of claim 1 to 4, wherein the first incubation time T1 is 17 to 27 hours, preferably about 24 hours.

6. Method according to any of claims 1 to 5, wherein the second incubation time T2 is 35 to 50 hours, preferably about 48 hours.

7. Method according to any of Claims 1 to 6, wherein the culture medium B comprises secretome in an amount of 0.5% to 3%, preferably about 1%.

8. Method according to Claim 2, wherein in step 2c), the loaded matrix is first incubated in standard culture medium A for about 30 min prior to incubating the loaded matrix in culture medium B for first the incubation time T1.

9. Method according to Claim 2 or 8, wherein washing step 2f) involves a first culture medium exchange step in which the culture medium B is exchanged with fresh standard culture medium A, and preferably involves at least a second culture medium exchange step in which previously added standard culture medium A is exchanged by fresh standard culture medium A.

10. Method according to Claim 9, in which the first and/or second culture medium exchange step is conducted by
i) transferring the matrix into a new container comprising fresh standard culture medium A and
ii) repeating step i) at least once, preferably at least twice, most preferably three times.

11. Method according to any of Claims 2 and 8 to 10, wherein the matrix is sterilized before being seeded with cells in step 2b) by exposing the matrix to a H₂O₂ plasma or a H₂O₂ containing atmosphere for either
- at least 1 minute, preferably at least 5 minutes, more preferably 5 to 20 minutes, at a temperature between 40°C and 50°C, or
- for at least 4 hours, more preferably about 8 to 12 hours, most preferably for about 10 hours, at a temperature below 40°C.

12. Method according to any of Claims 2 and 8 to 11, wherein the matrix is plasma-treated before being seeded with cells in step 2b) by exposing the matrix to a reactive gas plasma for at least 1 minute, preferably at least 5 minutes, more preferably about 10 minutes, at a temperature below 50°C.

13. Method according to any of Claims 2 and 8 to 12, wherein the matrix is loaded with at least 250'000 cells, preferably at least 500'000 cells, per cm³.

14. Method according to any of Claims 2 and 8 to 13, wherein the matrix is loaded with at least 1x10⁶ cells.

15. Method according to any of Claims 2 and 8 to 14, wherein the matrix consists of a basic polymer structure mainly or completely consisting of polylactic-acid that is preferably coated with collagen, preferably by covering the basic polymer structure with 1% collagen solution and subsequent freeze-drying.
